# FASCICULE DE BREVET EUROPEEN

(11) **EP 0 935 485 B1**
(45) Date de publication et mention de la délivrance du brevet: **17.08.2005**
(21) Numéro de dépôt: 98939460.6
(22) Date de dépôt: 28.08.1998
(51) Int. Cl.: A61N 5/06

(54) **DISPOSITIF DIFFUSEUR DE LUMIERE POUR LE TRAITEMENT PHOTODYNAMIQUE D'ORGANES**
LICHTDIFFUSIONSVORRICHTUNG ZUR PHOTODYNAMISCHEN BESTRAHLUNG VON ORGANEN
LIGHT DIFFUSING DEVICE FOR PHOTODYNAMIC TREATMENT OF ORGANS

(30) Priorité: 04.09.1997 FR 9711254
(43) Date de publication de la demande: 18.08.1999
(73) Titulaire: Medlight S.A., 1024 Ecublens (CH)
(72) Inventeur: THIELEN, Patrick, CH-1224 Genève (CH); WOODTLI, Alain, CH-2024 Saint-Aubin (CH)
(74) Mandataire: Besse, François
(86) Numéro de dépôt international: PCT/CH1998/000371
(87) Numéro de publication internationale: WO 1999/011323

(56) Documents cités:
- WO-A-90/00914
- WO-A-93/25155
- WO-A-96/07451
- WO-A-97/07735
- US-A- 5 536 265

## Description

### Domaine technique

La présente invention concerne un dispositif diffuseur de lumière utilisable notamment pour l'irradiation de tissus biologiques dans le cadre d'un traitement photodynamique, ce diffuseur comportant un conducteur de lumière en particulier une fibre optique ayant un tronçon d'extrémité dénudé jusqu'au coeur, ce tronçon d'extrémité étant entouré d'au moins une couche d'une substance diffusante ayant des propriétés de diffusion de la lumière circulant dans ledit conducteur de lumière.

### Technique antérieure

Dans le cadre du traitement photodynamique des lésions cancéreuses superficielles ou d'autres affections pathologiques d'organes du corps humain, la technique actuellement utilisée consiste à injecter, par voie intraveineuse, un sensibilisateur qui se localise de préférence dans les tissus néoplastiques. La portion de tissus malade contenant le photosensibilisateur est ensuite irradiée avec une lumière dont la longueur d'onde est déterminée en fonction du photosensibilisateur injecté. Cette lumière active le photosensibilisateur et induit une réaction chimique qui détruit les cellules. Cette irradiation se fait à l'aide de diffuseurs souples guidant de la lumière provenant généralement d'une source laser et diffusant cette lumière dans l'organe à traiter de façon contrôlée. L'intensité de la lumière émise par le diffuseur doit être homogène sur toute la surface à traiter pour éviter les problèmes liés à une surexposition ou à une sous-exposition. Une surexposition provoquée par une forte énergie lumineuse peut produire des dommages aux tissus sains sous-jacents ainsi qu'un échauffement local. Une sous-exposition peut conduire à une récidive de la maladie si certaines cellules ne sont pas tuées par le traitement.

On connaît déjà des diffuseurs de ce type, notamment le diffuseur de lumière décrit par le brevet américain US-A-5,536,265. Ce diffuseur comporte une fibre optique dont un tronçon d'extrémité a été dénudé jusqu'au coeur, une première couche d'une substance élastique transparente, une deuxième couche d'un matériau ayant des propriétés de diffusion de la lumière, cet ensemble étant contenu dans une pièce tubulaire transparente constituant une gaine de protection.

D'une part, ce diffuseur est de construction complexe. D'autre part, le tronçon d'extrémité de la fibre optique, qui a une longueur pouvant atteindre une dizaine de centimètres pour un diamètre de quelques centaines de microns, est extrêmement difficile à centrer à l'intérieur de la pièce tubulaire en raison de la souplesse de ces deux éléments. De ce fait, la deuxième couche faite d'un matériau ayant des propriétés diffusantes est irrégulière autour du coeur de la fibre dénudée qui constitue ce tronçon d'extrémité. Or les propriétés diffusantes sont liées notamment à l'épaisseur de matière diffusante entourant le coeur.

Pour obtenir une diffusion homogène de la lumière transmise par la fibre optique, il est essentiel que les particules diffusantes soient réparties de façon homogène et que l'épaisseur de la couche diffusante soit constante.

### Exposé de l'invention

La présente invention se propose de pallier les inconvénients de l'art antérieur en offrant un dispositif diffuseur répondant à ce but et dont la construction est simplifiée.

A cet effet, le dispositif selon l'invention est caractérisé en ce que ladite couche de substance diffusante de la lumière est constituée par une pièce tubulaire préfabriquée ayant des parois d'épaisseur constante, dans laquelle est engagé ledit tronçon d'extrémité de la fibre.

Selon une forme de réalisation avantageuse, ladite pièce tubulaire est réalisée en un matériau thermoformable dans lequel sont incorporées des particules diffusantes ayant la propriété de diffuser la lumière, ces particules étant réparties de façon homogène dans ledit matériau thermoformable.

De préférence, il comporte une couche d'un matériau transparent disposée entre la paroi extérieure du tronçon d'extrémité et la paroi intérieure de la pièce tubulaire.

De façon avantageuse, le dispositif peut comporter à son extrémité distale un miroir agencé pour renvoyer dans la fibre la lumière sortant par la surface d'extrémité du tronçon de fibre.

La pièce tubulaire est obtenue de préférence par extrusion d'un segment tubulaire de grande longueur en matériau thermoformable contenant des particules diffusantes, et par découpe d'un tronçon de longueur prédéterminée.

La pièce tubulaire ayant des propriétés de diffusion forme avantageusement la gaine de protection extérieure du tronçon de fibre optique.

### Description sommaire des dessins

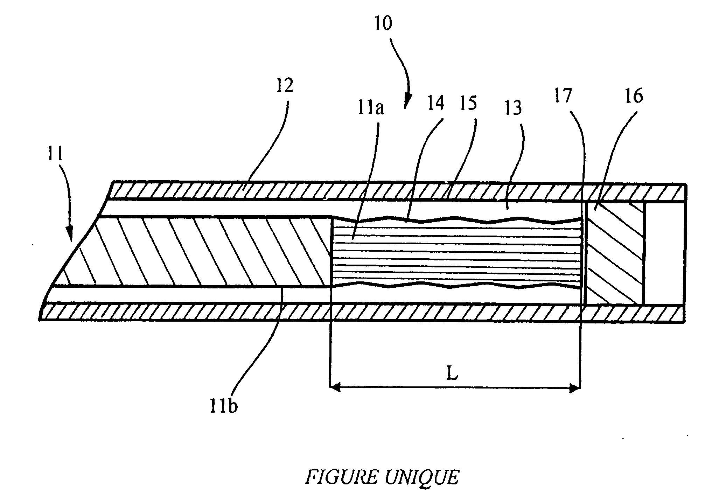

La présente invention sera mieux comprise en référence à la description d'un mode de réalisation préféré et au dessin annexé dans lequel la figure unique représente une vue en coupe de l'extrémité d'un dispositif diffuseur selon l'invention.

### Meilleure manière de réaliser l'invention

En référence à la figure, l'extrémité du dispositif diffuseur 10 se compose du tronçon d'extrémité d'une fibre optique 11, formée d'un coeur 11a entouré d'un manteau 11 b, logé dans une pièce tubulaire formant une gaine 12. Ce tronçon de la fibre 11 est maintenu en position dans la gaine 12 par une couche d'un matériau 13 disposée entre la paroi extérieure 14 du tronçon de fibre 11 et la paroi intérieure 15 de la gaine 12. Un miroir 16 logé dans la gaine 12 est plaqué contre la surface d'extrémité 17 du tronçon de fibre 11 et forme l'extrémité distale du diffuseur 10.

Pour fabriquer ce diffuseur 10, on procède tout d'abord à une première étape qui consiste à détruire le manteau 11b de la fibre 11 sur une longueur L correspondant à la longueur de la partie diffusante du diffuseur 10, afin de permettre à une partie de la lumière de s'échapper hors du coeur de la fibre. Lorsque cette partie du manteau a été éliminée, la partie du coeur 11a ainsi dénudée est rendue rugueuse.

L'obtention de cette rugosité peut être se faire de différentes manières telles que
- l'attaque par une substance chimique à l'aide d'un acide, d'une base ou d'un solvant,
- l'attaque mécanique par abrasion, sablage ou enlèvement de copeaux,
- l'attaque thermique au moyen d'air chaud, d'un outil chauffé ou d'un laser thermique, ou
- l'usinage au laser à l'aide d'un laser excimère.
   Le procédé utilisé doit permettre le contrôle de la rugosité de manière à ce que la quantité de lumière qui s'échappe ainsi du coeur 11 a de la fibre sur la longueur L corresponde au profil souhaité.

Lors d'une deuxième étape, le tronçon de fibre 11 dénudé est enfilé dans une gaine extérieure 12. Cette gaine est d'une part destinée à protéger la fibre de l'action d'agents extérieurs, tels que les produits chimiques utilisés pour la désinfection du diffuseur avant et après son utilisation, et des chocs mécaniques pouvant se produire lors des manipulations, et d'autre part, à homogénéiser la distribution angulaire de la lumière s'échappant de la partie rugueuse de la fibre et qui obéit aux phénomènes de diffraction et de réfraction.

Pour obtenir cette homogénéisation, la gaine est réalisée en une matière thermoplastique dans laquelle sont noyées des particules d'une substance diffusante, lesdites particules ayant pour but de diffracter et/ou de réfracter la lumière émise par le tronçon de fibre 11 et de la rendre homogène. La substance utilisée est par exemple le dioxyde de titane (TiO₂) qui est incorporé à la matière thermoplastique dans une proportion d'environ 1%. La préparation du mélange de la substance diffusante et de la matière thermoplastique peut se faire en relativement grande quantité et de façon industrielle afin d'obtenir une répartition homogène des particules. Ce mélange est ensuite extrudé et un segment de grande longueur de gaine est thermoformée de manière connue en soi.

La gaine 12 ainsi obtenue présente une épaisseur de matière diffusante constante et peut être réalisée avec une grande précision. Des tronçons de gaine utilisables pour la réalisation de dispositifs diffuseurs sont découpés dans le segment de grande longueur, ce qui permet également de garantir une constance des caractéristiques optiques des tronçons ainsi obtenus.

La fibre 11 est maintenue dans la gaine 12 à l'aide d'une couche 13 d'un matériau de liaison transparent introduit entre la paroi extérieure 14 du tronçon de fibre 11 et la paroi intérieure 15 de la gaine 12. Le matériau généralement utilisé est du caoutchouc silicone transparent car il présente une excellente transmission optique. Son indice de réfraction est inférieur à celui du coeur de la fibre et ses qualités mécaniques sont bien connues. Néanmoins l'utilisation de tout autre matériau souple transparent dont l'indice de réfraction est faible peut être envisagé. L'épaisseur de la couche 13 n'est pas un facteur clé dans la réalisation du diffuseur selon l'invention car une épaisseur d'environ 630 nm suffit à garantir l'effet guide d'onde recherché. Grâce à son faible indice de réfraction, cette couche joue le rôle de manteau pour le coeur 11a dénudé du tronçon d'extrémité de la fibre 11.

Le miroir 16, dont la face réfléchissante est plaquée contre la surface d'extrémité 17 du tronçon de fibre 11, permet de limiter les pertes de lumière à l'extrémité de la fibre en renvoyant dans la partie diffusante la lumière sortant par ladite surface d'extrémité 17.

L'avantage du diffuseur ainsi obtenu par rapport à celui qui fait l'objet du brevet américain cité en préambule est que l'épaisseur de la matière diffusante est toujours constante car la fabrication d'une gaine par extrusion d'une matière thermoplastique est bien maîtrisée. Dans le diffuseur de l'art antérieur, l'épaisseur de matière diffusante dépend du centrage de la fibre dans la gaine extérieure, ce qui est très difficile à contrôler, étant donné que la fibre et la gaine extérieure sont souples et que le centrage doit être obtenu sur une longueur pouvant aller jusqu'à 10 cm. La probabilité que la fibre touche la gaine extérieure en un point est grande, ce qui a pour conséquence d'avoir une épaisseur de matière diffusante très faible en ce point de contact, donc une moins bonne diffusion de la lumière. Dans le diffuseur selon l'invention, le centrage de la fibre dans la gaine n'est pas critique car l'épaisseur de la couche transparente 13 n'influence pas de façon significative la répartition de la lumière. De plus la construction de ce diffuseur est simplifiée car la gaine 12, qui est obtenue de façon standard, sert de gaine de protection extérieure tout en étant diffusante et permet ainsi la suppression d'une couche intermédiaire.

Dans d'autres variantes de réalisation on peut prévoir que la gaine soit directement thermoformée autour de la fibre. On peut également prévoir de remplacer la couche de matériau transparent par une couche d'air et de fixer la fibre dans la gaine uniquement par des points de colle régulièrement espacés sur la paroi intérieure de la gaine.

## Revendications

1. Dispositif diffuseur de lumière utilisable notamment pour l'irradiation de tissus biologiques dans le cadre d'un traitement photodynamique, ce diffuseur (10) comportant un conducteur de lumière comprenant une fibre optique (11) ayant un tronçon d'extrémité dénudé jusqu'au coeur (11a), ce tronçon d'extrémité étant entouré d'au moins une couche d'une substance diffusante ayant des propriétés de diffusion de la lumière circulant dans ledit conducteur de lumière, **caractérisé en ce que** ladite couche de substance ayant des propriétés de diffusion de la lumière est constituée par une pièce tubulaire préfabriquée (12) ayant des parois d'épaisseur constante, dans laquelle est engagé ledit tronçon d'extrémité de la fibre (11).

2. Dispositif selon la revendication 1, **caractérisé en ce que** ladite pièce tubulaire (12) est réalisée en un matériau thermoformable dans lequel sont incorporées des particules diffusantes ayant la propriété de diffuser la lumière, ces particules étant réparties de façon homogène dans ledit matériau thermoformable.

3. Dispositif selon la revendication 1, **caractérisé en ce qu'**il comporte une couche (13) d'un matériau transparent disposée entre la paroi extérieure (14) du tronçon d'extrémité (11) et la paroi intérieure (15) de la pièce tubulaire (12).

4. Dispositif selon la revendication 1, **caractérisé en ce qu'**il comporte à son extrémité distale un miroir (16) agencé pour renvoyer dans la fibre la lumière sortant par la surface d'extrémité (17) du tronçon de fibre (11).

5. Dispositif selon la revendication 2, **caractérisé en ce que** la pièce tubulaire (12) est obtenue par extrusion d'un segment tubulaire de grande longueur en matériau thermoformable contenant des particules diffusantes, et par découpe d'un tronçon de longueur prédéterminée.

6. Dispositif selon la revendication 5, **caractérisé en ce que** le matériau extrudé comporte environ 1 % en poids de particules diffusantes.

7. Dispositif selon la revendication 1, **caractérisé en ce que** la pièce tubulaire (12) ayant des propriétés de diffusion forme une gaine de protection extérieure du tronçon de fibre (11).

## Claims

1. Light diffuser device that can be used especially for the irradiation of biological tissues within the context of photodynamic treatment, this diffuser (10) comprising a light conductor consisting of an optical fibre (11) having a tip section stripped down to the core (11a), this tip section being surrounded by at least one layer of a scattering substance having the property of scattering the light travelling along the said light conductor, **characterized in that** the said layer of substance having light-scattering properties consists of a prefabricated tubular part (12) having walls of constant thickness, into which part the said tip section of the fibre (11) is engaged.

2. Device according to Claim 1, **characterized in that** the said tubular part (12) is made of a thermoformable material into which scattering particles having the property of scattering light are incorporated, these particles being uniformly distributed in the said thermoformable material.

3. Device according to Claim 1, **characterized in that** it includes a layer (13) of a transparent material placed between the outer wall (14) of the tip section (11) and the inner wall (15) of the tubular part (12).

4. Device according to Claim 1, **characterized in that** it includes, at its distal end, a mirror (16) designed to reflect the light emanating via the end surface (17) of the section of fibre (11) back into the fibre.

5. Device according to Claim 2, **characterized in that** the tubular part (12) is obtained by extruding a long tubular segment made of thermoformable material containing scattering particles and by cutting off a section of predetermined length.

6. Device according to Claim 5, **characterized in that** the extruded material contains about 1% by weight of scattering particles.

7. Device according to Claim 1, **characterized in that** the tubular part (12) having scattering properties forms an external protective sheath for the section of fibre (11).

## Patentansprüche

1. Lichtverteilungsvorrichtung, die insbesondere für die Bestrahlung von biologischen Geweben im Rahmen einer photodynamischen Behandlung verwendet werden kann, wobei diese Verteilungsvorrichtung (10) einen Lichtleiter besitzt, umfassend eine optische Faser (11) mit einem bis zum Kern (11a) freigelegten Endabschnitt, wobei dieser Endabschnitt von mindestens einer Schicht einer Verteilungssubstanz umgeben ist, die Eigenschaften zur Verteilung des in dem Lichtleiter zirkulierenden Lichts besitzt, **dadurch gekennzeichnet, dass** die Schicht der Substanz, die Lichtverteilungseigenschaften hat, von einem vorgefertigten röhrenförmigen Teil (12) gebildet ist, der Wände mit konstanter Dicke aufweist und in den der Endabschnitt der Faser (11) eingesetzt ist.

2. Vorrichtung nach Anspruch 1, **dadurch gekennzeichnet, dass** der röhrenförmige Teil (12) aus einem wärmeformbaren Material hergestellt ist, in das Verteilungspartikel eingesetzt sind, die die Eigenschaft haben, dass sie das Licht verteilen, wobei diese Partikel homogen in dem wärmeformbaren Material verteilt sind.

3. Vorrichtung nach Anspruch 1, **dadurch gekennzeichnet, dass** sie eine Schicht (13) aus einem transparenten Material umfasst, die zwischen der Außenwand (14) des Endabschnitts (11) und der Innenwand (15) des röhrenförmigen Teils (12) angeordnet ist.

4. Vorrichtung nach Anspruch 1, **dadurch gekennzeichnet, dass** sie an ihrem distalen Ende einen Spiegel (16) umfasst, der derart angeordnet ist, dass er das über die Endfläche (17) des Faserabschnitts (11) austretende Licht in die Faser zurückschickt.

5. Vorrichtung nach Anspruch 2, **dadurch gekennzeichnet, dass** der röhrenförmige Teil (12) durch Extrusion eines röhrenförmigen Segments von großer Länge aus einem wärmeformbaren Material, das Verteilungspartikel enthält, und durch Ausschneiden eines Abschnitts mit vorbestimmter Länge erhalten wird.

6. Vorrichtung nach Anspruch 5, **dadurch gekennzeichnet, dass** das extrudierte Material ungefähr 1 Gew.-% Verteilungspartikel umfasst.

7. Vorrichtung nach Anspruch 1, **dadurch gekennzeichnet, dass** der röhrenförmige Teil (12), der Verteilungseigenschaften hat, eine äußere Schutzhülle für den Faserabschnitt (11) bildet.
